# EUROPEAN PATENT SPECIFICATION

(11) **EP 0 644 896 B1**
(45) Date of publication and mention of the grant of the patent: **27.11.2002**
(21) Application number: 93916913.2
(22) Date of filing: 01.07.1993
(51) Int. Cl.: C07K 16/06, C07K 16/42, A61K 38/02, A61K 38/04

(54) **PEPTIDES REPRESENTING ANTIGENIC EPITOPES OF IgE PRESENT ON B CELL BUT NOT BASOPHIL SURFACE**
ANTIGENE EPITOPE VON IgE REPRÄSENTIERENDEN PEPTIDEN AUF DER B-ZELL-OBERFLÄCHE ABER NICHT AUF OBERFLÄCHEN VON BASOPHILEN
PEPTIDES REPRESENTANT DES EPITOPES ANTIGENIQUES DE L'IgE PRESENTS SUR LA SURFACE DES LYMPHOCYTES B MAIS NON SUR CELLE DES CELLULES BASOPHILES

(30) Priority: 11.03.1993 US 31398
(43) Date of publication of application: 29.03.1995
(73) Proprietor: TANOX BIOSYSTEMS, INC., Houston, TX 77025 (US)
(72) Inventor: CHANG, Tse, Wen, Houston, TX 77005 (US)
(74) Representative: Klunker . Schmitt-Nilson . Hirsch
(86) International application number: US9306278
(87) International publication number: WO94020533

(56) References cited:
- WO-A-89/06138
- WO-A-91/11456
- US-A- 5 091 313
- Bio/Technology, Volume 9, issued January 1991, F.M. DAVIS et al., "An Epitope on Membrane-Bound But Not Secreted IgE: Implications in Isotype-Specific Regulation", pages 53-56, see entire document.
- The EMBO Journal, Volume 1, Issue No. 9, issued 1982, N. ISHIDA et al., "The Nucleotide Sequence of the Mouse Immunoglobulin Epsilon Gene: Comparison with the Human Epsilon Gene Sequence", pages 1117-1123, see entire document.
- Nature, Volume 296, issued 01 April 1992, H.L. CHENG et al., "Structure of Genes for Membrane and Secreted Murine IgD Heavy Chains", pages 410-415, see entire document.
- Molecular Immunology, Volume 25, issue No. 2, issued 1988, R. SITIA et al., "The Control of Membrane and Secreted Heavy Chain Biosynthesis Varies in Different Immunoglobulin Isotypes Produced by a Monoclonal B Cell Lymphoma", pages 189-197, see entire document.
- Nature, Volume 307, issued 02 February 1984, F. BLATTNER et al., "The Molecular Biology of Immunoglobulin D", pages 417-422, see entire document.

## Description

The present invention relates to a peptide representing the ε.mb/ec segment of a dog or peptides having the same immunological properties as said peptide, a vaccine comprising said peptide(s), the use of said peptide(s) for the preparation of a medicament for treating a dog afflicted with allergy, and a nucleotide encoding said peptide.

### Background

The immediate-type hypersensitivities, such as extrinsic asthma, hay fever, and allergic responses to certain food or drugs, are mediated primarily by immunoglobulin E (IgE). In an IgE-mediated allergic response, the allergen binds to IgE on the surface of mast cells and basophilic leukocytes (basophils). This binding causes a crosslinking of the IgE molecules and hence, the underlying receptors for the Fc portion of IgE (FcεR), and thereby triggers the release of pharmacologic mediators, such as histamine, the slow-reacting substance of anaphylaxis, and serotonin. The release of these mast cell and basophil products causes the various pathological manifestations of allergy.

IgE is produced by a particular class of B lymphocytes (B cells), which also bear IgE on their surface. In individuals sensitized to specific allergens, the allergen-specific IgE is produced by B cells continuously.

IgE binds to the receptors for the Fc of IgE (FcεR) on the surface of basophils and mast cells very strongly. The association constant, Ka, is in the neighborhood of 1 x 10¹⁰ liter/mole and the "off" time is more than 20 hours. The very strong and stable association of IgE with Fc∈R means that IgE is virtually always present on these cells. An immunotherapeutic agent targeting the IgE on B cells must not react with the IgE on basophils and mast cells. Antibodies which react with the IgE isotype will cross-link IgE and the underlying FcεR on basophils and mast cells and, when administered *in vivo*, will induce systemic release of pharmacologic mediators, leading to anaphylaxis.

The development of monoclonal antibodies that recognize an antigenic epitope present on the IgE on B cells, but not the IgE on basophils, was described in US-A-5 422 258, filed 7/29/88. These antibodies also bind to the secreted form of IgE. In addition, the method of using such antibodies for treating allergy, either in the form of plain antibodies or toxin-conjugated antibodies, was described. The plain antibodies can cause the pharmacological mechanism of antibody-dependent cellular cytotoxicity (ADCC), and the toxin-conjugated antibodies can directly cause cytolysis. Both of these mechanisms can lead to the depletion of IgE-bearing B cells without harming the basophils and mast cells. The IgE epitopes present on B cells but absent on basophils are termed ige.bl epitopes (bl stands for B lymphocytes).

If a vaccine was developed which induced production of antibodies which could specifically target IgE-bearing B cells, without targeting any other cells, such a vaccine would be useful in treating allergy and related hypersensitivities. It has been observed that IgE is not needed for normal health, except perhaps for combatting parasites. Thus, depleting all IgE-bearing B cells would not produce any adverse effects.

WO 89/06138 and WO 91/11456 disclose ige.bl epitopes, and in particular the amino acid sequence of the mb/ec segment of human IgE (ε.mb/ec segment), as well as the nucleotide sequence coding therefor. These documents also disclose IgE-mediated allergy treatment and immunization on the basis of the respective peptides. The amino acid sequence of the dog ∈.mb/ec segment and the nucleotide sequence coding therefor are not disclosed.

### Summary of the Invention

The invention relates to antigenic epitopes (and peptides representing these epitopes) which are present on dog B cell membrane-bound immunoglobulins but not on the secreted, soluble form of the immunoglobulin. The invention also pertains to compositions exploiting these newly discovered epitopes for therapeutic and diagnostic purposes.

The present invention provides a peptide representing the ε.mb/ec segment of a dog, the peptide having the sequence shown in SEQ ID NO:3, and a peptide having a sequence wherein the sequence shown in SEQ ID NO:3 is modified by deletion, insertion or substitution of one or more amino acids which do not detract from the immunological property of the peptide to elicit antibodies which react specifically with membrane-bound immunoglobulin ε of a dog.

The present invention also provides the use of a peptide having the sequence shown in SEQ ID NO:3 or of a peptide having a sequence wherein the sequence shown in SEQ ID NO:3 is modified by deletion, insertion or substitution of one or more amino acids which do not detract from the immunological property of the peptide to elicit antibodies which react specifically with membrane-bound immunoglobulin ε of a dog for the preparation of a medicament for treating a dog afflicted with allergy.

Further, the present invention provides a vaccine comprising a peptide having the sequence shown in SEQ ID NO:3 or a peptide having a sequence wherein the sequence shown in SEQ ID NO:3 is modified by deletion, insertion or substitution of one or more amino acids which do not detract from the immunological property of the peptide to elicit antibodies which react specifically with membrane-bound immunoglobulin ε of a dog.

In addition, the present invention provides a nucleotide consisting of the sequence of the exons of SEQ ID NO:2 or the corresponding or complementary mRNA sequence.

B cells express on their surface antibody molecules, which serve as receptors for antigens during immunological induction. The membrane-bound immunoglobulins differ from the secretory, soluble immunoglobulins synthesized by the same cells in that they have an extra peptidic piece that anchors them onto the B cell surface.

All ten membrane-bound immunoglobulins on B cells from different species, for which amino acid sequences have been determined, have extra isotype-specific regions that anchor the immunoglobulins to the membrane. These peptidic regions have lengths ranging from 41 to 72 amino acids, and can be divided into three segments in terms of locations in relation to the plasma membrane. The middle segments of 25 hydrophobic and uncharged amino acid residues are in the membrane lipid bilayer. The C terminal hydrophilic segments of 3-28 amino acid residues are intracellular.' The segments toward N-terminus contain 13 to 27 amino acid residues and are highly acidic and hydrophilic. They are located on the extracellular surface of the plasma membrane.

The length and the hydrophilic and highly charged nature of the extracellular segment indicates that this segment is exposed and accessible to antibodies. These antigenic epitopes located on the extracellular segment of membrane-bound region of immunoglobulin heavy chains can be generally designated mb/ec, and the mb/ec segment of IgE is designated as the ε.mb/ec segment.

The present invention pertains to the discovery of the ε.mb/ec epitope of a dog and the peptide representing it, and the use of such peptide for allergy treatment and for generating monoclonal and polyclonal antibodies which target the ε.mb/ec epitopes on B cells.

### Detailed Description of the Invention

### 1. Unique Antigenic Epitopes of IgE as Targets of Immunotherapeutic Agents

IgE is present on three cell types in the body: IgE-producing B cells, mast cells, and basophils. If an antigenic epitope of IgE is present on B cells and not on basophils and mast cells, these epitopes (defined as ige.bl) are virtually unique cell surface markers of IgE-bearing B cells. These markers provide targets for several types of monoclonal or polyclonal antibody-based therapy for IgE-mediated allergic diseases, and provide a means to differentiate B cells producing IgE from B cells producing other isotypes.

### 2. Anchoring Peptidic Piece of B Cell Membrane-bound Immunoglobulins

The amino acid sequence data of the ten membrane-bound immunoglobulins from several species have been determined. *See* Ishida, N. et al., EMBO J., 1:1117 (1982); Steen, M. L. et al., J. Mol. Biol., 177:19-32 (1984); Rogers, J. et al., Cell, 26:19-27 (1981); Yamawaki-Kataoka, Y. et al., Proc. Natl. Acad. Sci., USA, 79:2008-2012 (1982); Kamaromy, M. et al., Nuc. Acids Res., 11:6775-6785 (1983); Rogers, J. et al., Cell, 20:303-312 (1980); Bernstein, K. E., J. Immunol. 132:490-495 (1984); Cheng, H. et al., Nature, 296:410-415 (1982). These sequences indicate certain common features of the plasma membrane-bound peptidic piece. The peptidic anchor piece has three segments which are distinguishable based upon their locations in relation to the plasma membrane. Even though these peptidic pieces are short, ranging from 41 to 72 amino acid residues, and have often been referred to as the "membrane-bound domain", the peptides are not entirely in the membrane lipid bilayer. In fact, only 25 amino acid residues (which are the largely hydrophobic residues and threonine and serine) located in the middle part of the peptides, are in the lipid bilayer. The C-terminal, hydrophilic segments of 3 to 28 amino acid residues are located on the cytoplasmic side of the membrane. The segments toward the N-terminus, which are connected to the third or fourth constant domains of the immunoglobulin heavy chains (CH₃ or CH₄) are very hydrophilic and are on the extracellular side of the plasma membrane.

The shortest of the extracellular segments of the membrane-bond pieces of the immunoglobulins (which are the mouse and rabbit µ chains) have 13 amino acid residues. The mb/ec segments of all immunoglobulins contain high proportions of charged amino acid residues, and are almost entirely acidic residues. The proportions of charged amino acid residues and polar hydrophilic residues account for very high percentages of the amino acid composition of the mb/ec segment. These parameters indicate that all the mb/ec segments are exposed and long enough to be accessible by antibodies. Studies of the evolution of immunoglobulin heavy chains indicate the ε and γ chains are more related to each other (had more recent common ancestry) than to other chains (Lin, L. C. and Putnam, F. W., Proc. Natl. Acad. Sci. USA, 1981). In addition, the heavy chains evolved before the various mammals species, such as mice, rats, and humans, evolved. Thus, among the ten mb/ec segments that have been determined, it is probably the murine or the rat ε.mb/ec that the human and dog ε.mb/ec will be most related. The murine or rat ε.mb/ec segment has 19 amino acid residues, among them 8 Glu and 2 Asp residues.

### 3. Determining the Amino Acid Sequence of an ε.mb/ec Segment, exemplary shown by the Human ε.mb/ec Segment. The claims, however, are not drawn to the Human ε.mb/ec Segment.

A number of well established procedures can be applied to determine the DNA sequence corresponding to the human ε.mb/ec segment. In one approach, the starting point is the mRNA preparation of a human myeloma cell line which expresses IgE on the surface, for example, SK007 cells. With the mRNA preparation, one can establish a cDNA library employing cloning vectors with λ phage or plasmids. A preferred method for constructing the cDNA library is with the cDNA Library Construction System Kit - Librarian I; developed and commercialized by Invitrogen (San Diego, CA). A stepwise detailed instruction manual is provided for RNA isolation from cells, reverse transcription, second strand synthesis, linker ligation, agarose gel sizing of cDNA, electroelution to purify cDNA vector ligation, and transformation of E. coli. The vector used in this library is pDCM8.

In the screening of the cDNA library for clones containing the ε.mb/ec segment, several probes can be used. The library can be screened with a DNA probe a, which is a 1.1 kb long U266 cDNA covering most of length of ε mRNA (no membrane-bound secreted and membrane-bound forms can be distinguished by using additional probes). A probe b is developed by taking advantage of the likelihood that the end of the CH₄ domain is truncated in the human ε chain of the membrane-bound form. The truncation occurs where gene segments of CH₄ domain and the membrane-bound domain are translocated. The loss of C-termini occur with the membrane- bound forms of immunoglobulins, including ε and λ, which contain CH₄ domains. From the published information on the nucleotide sequence of the human ε CH₄ domain, the most probable splicing donor site is intracodon GT, 71 bp 5' of the termination codon TGA. Another GT, which is not intracodon and less likely a splicing donor site, is closer to the terminus (24 bp 5' to the termination codon). Probe b will react with secreted form of ε chain gene and not the membrane-bound form of the ε chain gene.

The design of a probe c is based on the finding that the transmembrane segment of the membrane-bound domain (mb/tm segment) is very conserved among all the immunoglobulin genes so far sequenced. There is a segment of peptide and its corresponding coding DNA within this mb/tm segment that is nearly identical among all immunoglobulins.

A probe d, which represents a segment upstream to the most possible splicing donor site, GT, consists of 36 bp. This probe should react with the ε chain gene of both the secreted and membrane-bound forms. The location of probes a, b, c, and d, in schematic form, is shown in WO 91/11456.

Table 1 summarizes the pattern of reactivities of clones containing ε genes of secreted or membrane-bound immunoglobulins with the four probes.

**Table 1.**

| The reactivity of ε gene-containing cDNA clones with probes a, b, c, and d. | | |
|---|---|---|
| | ε secreted | ε Membrane-bound |
| Probe a | + | + |
| Probe b | + | - |
| Probe c | - | + |
| Probe d | + | + |

The library size needed to clone the membrane-bound ε chain depends on how abundant the mRNA is. Assuming secreted IgE comprises 0.1% of the SK007 poly A⁺ RNA, the library size should be about 5,000 independent recombinant clones, in order to have a 99% possibility of isolating a positive clone. In IgE-producing rat immunocytoma IR2 and IR162 cells, mRNA for the membrane-bound form of ε chain was found to be more than 2% of that of the secreted form. Assuming this ratio of membrane-bound/secreted forms of ε chain holds true for the human IgE-producing SK007 cells, the cDNA library size needed to isolate the membrane-bound ε chain is about 250,000. In a preferred procedure, a larger number of clones, about 1,000,000, are screened.

An alternative to the conventional approach of establishing a cDNA library and screening the clones representing the cellular mRNA species, is to amplify the mRNA to produce high proportions of their corresponding DNA. The resulting DNA can then be purified by gel electrophoresis and subjected to sequence analysis. The methodology, referred to as polymerase chain reaction (PCR) amplification, has been established in the past few years and a complete system, including reagents and equipment, have been commercialized. One preferred system is provided by Perkin Elmer Cetus (Norwalk, CT). The reagent kit is the GeneAmp DNA Amplification Reagent Kit and the equipment is the DNA Thermal Cycler.

Some of the specific reagents used in this approach are the same as used for the cDNA library cloning. Since no sequence in the membrane-bound segment of the ε chain has been determined, the strategy is to amplify both the secreted and membrane-bound forms of the ε chains. Two primers are to be used, one is oligo.dT (25-30-mers) and one is the oligomer corresponding to probe d. Probe d is located 5' to the most possible splicing donor site and therefore primes both the secreted and membrane-bound forms of ε mRNA and DNA. After sufficient amplification, the two populations of DNA fragments are resolved by gel electrophoresis. The secreted form of the ε chain can be distinguished by its reactivity with probe b. The purified DNA is then subjected to DNA sequencing.

PCR amplification seems to be more efficient procedure than cDNA cloning for mRNA poorly represented in the poly A⁺ RNA pool. The U266 ε chain cDNA can be used to work out some preliminary annealing conditions between template DNA and the oligo-primers.

Another approach for obtaining a DNA clone containing genes encoding the membrane-bound segments is to screen the human genomic DNA library. The human genomic DNA library is readily available. A preferred source is the library constructed using human lung fibroblast WI38 cells provided by Stratogene (La Jolla, CA). The genes are in λ vector and the inserted DNA have average sizes of 15K bp. Identification of the clones can be achieved by hybridization with U266 cDNA. The location of the gene segment corresponding to the membrane-bound region can be determined by using a probe prepared from the homologous mouse gene of the transmembrane segment (probe c, discussed above). The sequence of the membrane-bound segment is then determined, and this sequence is shown in WO 91/11 456.

Nucleotide sequence of genomic DNA encompassing the encoding segments for the membrane anchoring peptide of human membrane bound ε chain was determined as described above. The assignment of the exons was made by identifying the nucleotides for splicing donors and acceptors, and by comparing them to the published homologous sequences of mouse membrane-bound ε chain, as well as to immunoglobulins of other classes. Two exons were found to exist.

Human ε.mb/ec peptide is identified as the first fourteen amino acids encoded by membrane exon I. This precedes a stretch of about 25 hydrophobic amino acids which are the transmembrane region. Two possible structures of ε.mb/ec are shown in WO 91/11456, and the monomeric structure is SEQ ID NO:1.

As described more fully below, the ε.mb/ec peptides (from both humans and other species, the present invention being concerned with the ε.mb/ec peptide of a dog) can be used to elicit antibodies which react specifically with membrane-bound immunoglobulin ε. For this purpose, the peptides can be chemically synthesized by standard techniques of protein synthesis. A preferred method for synthesizing the peptides is with the RaMPS system (DuPont, Wilmington, DE), which applies Fmoc chemistry. Alternatively, the proteins can be biosynthesized by employing oligodeoxynucleotides encoding the peptides.

As immunogens or vaccines, the peptides may be used in either the monomeric or dimeric structural forms shown in WO 91/11456. In addition, modified peptides having substantial immunological equivalency can be used. For example, the peptide amino acid sequence shown above (or described below for dogs) can be modified by deletion, insertion or substitution of one or more amino acids which do not essentially detract from the immunological properties of the peptide. The peptides can also be used as polymers where the amino acid sequence shown above, or equivalent sequence, is the polymer repeat unit. Subject matter of the present invention are the dog peptide and modified peptides having the modifications mentioned above.

### 4. Sequencing the Dog ε.mb/ec Segment.

A dog genomic library was screened using radioactive mouse IgE cDNA probes covering the membrane and constant regions. A phage clone was selected as a positive and the phage DNA was purified. On restriction digestion and Southern hybridization analyses, a Bam HI fragment (size ∼ 2.6Kb) was identified by using a radioactive human membrane IgE probe. A much larger (7.5-8Kb) XhoI fragment lit up with the constant probe. The BamHI fragment was found to be part of the larger XhoI fragment. Hence, the XhoI fragment should contain both the constant and membrane regions.

Further characterization of the DNA was simplified by subcloning and restriction enzyme mapping the above fragments into known plasmid vectors, and then sequencing them via the Sanger di-deoxy method, using ³²P and ³⁵S labelled nucleotides. This enabled the identification of the membrane IgE region (SEQ ID NO:3), which was confirmed by comparing with the known mouse membrane sequence. The extracellular. membrane-bound domain (based on a plot of hydrophobicity/hydrophilicity) is the first 15 amino acids shown in SEQ ID NO:3. This region is fairly hydrophobic, and abuts a hydrophilic region. Using a similar approach, the constant region was identified. This was compared to the chimpanzee IgE constant region sequence for confirmation.

### 5. Producing a Cell Line which Secretes Dog IgE.

A chimeric heavy chain vector was next constructed using the heavy chain variable gene of BAT123 (an anti-HIV-1 mouse monoclonal antibody which is described in US-A-5 854 400, WO 88/09181) and the 7.5Kb XhoI genomic fragment, (containing the CH₁, CH₂, CH₃, CH₄, M₁ and M₂ region of Dog IgE and a 3' untranslated region), as the constant chain region. This vector was co-transfected along with the BAT123 mouse/human chimeric κ (which is described in WO 88/09181) light chain vector into NSO cells. Using 50 µg supercoiled DNA of the heavy chain and 10 µg linear DNA of the light chain, 32 x 10⁶ cells were transfected. The 11 x 10⁶ viable cells recovered were plated out in 96 well plates at a density of one million cells per plate. These were screened using solid phase anti-hκ and HRP conjugated AB19-4 (which is an anti-idiotype antibody).

Thirty-seven wells out of the 11 plates showed cell outgrowth. Twenty wells out of 37 showed an O.D. reading > 0.05 by the screening procedure outlined above. After the MTT assay, cell lines DE1 and DE10 were generated which showed secreted Ig levels of 2 µg/10⁶ cells in 24 hours. Expansion of DE1 was carried out in T-100 medium. Purification of 200 ml of supernatant, using an AB19-4 affinity column, yielded 700 µg of purified immunoglobulin. A reducing SDS-PAGE gel showed a light chain band identical to SE44 IgE and a slightly larger heavy chain band, confirming the presence of a secreted immunoglobulin with a BAT123 light chain. One liter and 3 liter spinner flasks were begun in T-100 medium, and 3.8 liters of supernatant was quantified by ELISA, and then prepared for purification.

The purified material could be used as an immunogen to generate monoclonal or polyclonal antibodies against Dog IgE, using the techniques described below.

### 6. Developing Antibodies to the ε.mb/ec Segment.

The ε.mb/ec peptide can be used in the immunization of animals to prepare polyclonal and monoclonal antibodies. They can also be used to screen for specific monoclonal antibodies or characterize specific polyclonal antibodies. They can also be used to purify monoclonal and polyclonal antibodies.

In the process of preparing for monoclonal antibodies specific for ε.mb/ec peptide, it is not necessary to use the ε.mb/ec peptide in both immunization and antibody identification. For example, in immunizing mice for preparing immune spleen cells for fusion with myeloma cells, the immunogen may be the membrane-bound IgE isolated from plasma membrane of IgE-bearing myeloma cells, such as SK007 cells. The immunogen may also be the IgE-bearing myeloma cells themselves. The immunogen can also be the purified dog IgE described above.

For using the synthetic ε.mb/ec peptide as the immunogen, it is more effective to conjugate the peptide to a protein carrier. A preferred protein carrier is keyhole limpet hemocyanin (KLH). If the peptidic segment lacks a lysine residue or if the lysine residue is in the middle part of the segment, it is desirable to add a lysine residue at the C-terminal end. Because the N-terminus already has an α-amino group, the modified synthetic peptidic will have two amino groups for linking.

Multiple molecules of peptides can be conjugated to each molecule of the carrier protein. With KLH, a preferred molar ratio for peptide/KLH is 10. The method of conjugation is very well established. Cross-linkers such as glutaraldehyde or bis (sulfosuccinimidyl) suberate or disulfosuccinimidyl tartarate (Catalogue #21579, 20591, Pierce Chemical Co., Rockford, IL) have been used. A preferred cross-linker is the latter.

The immunogen, such as the KLH conjugate, can be used to immunize rabbits, goats, rats, or mice to prepare polyclonal antibodies specific for the ε.mb/ec peptide. Lympocytes from the spleen or lymph nodes of immune mice and rats can also be taken to prepare hybridomas secreting monoclonal antibodies specific for the ε.mb/ec peptide. A preferred protocol to prepare the monoclonal antibodies is to fuse immune spleen cells of mice with non-secreting mouse myeloma cells, such as NS-1 or SP2/0 cells using polyethylene glycol.

In one immunization protocol for mice, 50 µg of the peptide-KLH conjugate in complete Freund's adjuvant is injected subcutaneously into each mouse for priming. Two and four weeks later, the same amounts of antigen are given s.c. in incomplete Freund's adjuvant. At about the six week time point, the fourth antigen injection is given i.p. in saline. Mice are sacrificed 4 days after the last injection and the spleens are taken for preparing single cell suspension for fusion with myeloma cells. A similar protocol can also be used for immunization with purified native human membrane-bound IgE (having attached membrane anchor domain) isolated from the plasma membrane of IgE-bearing human myeloma cells, such as SK007 cells. When human IgE-bearing cells are used as the immunogen, 1x10⁷ cells are injected i.p. at two week intervals.

The fusion procedure with polyethylene glycol and other various procedures concerning cloning and hybridoma culturing have been well established, and the preferred protocol is the same as described by Hudson, L. and Hay. F.C., (Practical Immunology, 2nd edition, pp. 303-313, Blackwell Publishing Co., Boston). The screening of hybridomas for monoclonal antibodies, or the identification of polyclonal antibodies or the identification of polyclonal antibodies reactive with ε.mb/ec peptide can be performed with enzyme linked immunosorbent assays (ELISAs), using the synthetic ε.mb/ec peptide as the solid phase antigen. An alternative solid phase antigen is the conjugate of ε.mb/ec peptide with a different carrier protein such as bovine serum albumin different from that used in the immunogen. Further characteristics of the monoclonal and polyclonal antibodies would be that they bind to an ε.mb/ec peptide, or to IgE-bearing B cells, but do not bind to soluble IgE, or to IgE on basophils or mast cells (as measured by a histamine release assay).

### 7. Using the ε.mb/ec Peptides for Animal Treatment.

The claims are not directed to methods for animal treatment, but to products for use in such methods.

The peptides can be tested in animal model systems to determine if they are suitable for allergy treatment in humans. In particular, the peptides of the invention can be used to treat dogs which are afflicted with allergies or other immediate-type hypersensitivities; for example, the dog ε.mb/ec peptide can be used to treat dog allergies or hypersensitivities. Similarly, the corresponding ε.mb/ec peptides from cats, horses, or other mammals, could be used to treat these animals.

When used to treat animals, the ε.mb/ec peptides, with a suitable carrier such as a recombinant protein antigen from rabies virus or from feline leukemia virus, are administered to the animal in a suitable pharmaceutical vehicle. Subcutaneous or intra-muscular injection is preferred as a route of administration. The dosage of peptide administered can vary widely depending on the size of the animal, but should be sufficient to create an immunogenic response in the animal. This would generally be in the range of about 10-100 µg/kg of animal body weight.

### 8. Animal Model Experiments.

One could also test the use of ε.mb/ec peptides in vaccination in a mouse model system, as described below. Mice are not known to develop allergic symptoms naturally. However, for demonstrating the pharmacologic mechanisms of the intended therapy in regard to the depletion of IgE-bearing B cells and IgE, the mouse can serve as an excellent model.

The *ε.*mb/ec segment of the mouse has already been sequenced. Ishida, N. et al., EMBO J. 1:1117-1123 (1982). The 19 amino acid residue peptide is shown in that publication.

The ε.mb/ec peptide is synthesized in several forms, including one that has extra Leu-Lys residues at the C-terminus. The peptide or its KLH conjugate could be used as the essential vaccine component, to immunize mice. One then examines whether the vaccine induces antibodies which target the IgE-bearing B cells, by examining the following questions:
(a) Does the total IgE in circulation decline?
(b) Does the number of IgE-bearing B cells decline?
(c) Does the density of IgE on the surface of basophils decline?
(d) Do IgM and IgG specific for ε.mb/ec peptide cause different effects? The purpose of this question is to address the effects of antibody dependent cellular cytotoxicity (ADCC) in the depletion of IgE-bearing B cells. IgG, not IgM, is known to mediate ADCC.

### 9. Diagnostic Uses for the Antibodies of the Invention.

Antibodies against ε.mb/ec epitopes can be used to identify and enumerate IgE-bearing lymphocytes in a fluid sample, such as serum. Thus, the various antibodies which target the ε.mb/ec epitope of a dog can be used diagnostically in dogs for such identification and enumeration of its IgE-bearing B lymphocytes.

For this purpose, antibodies can be used in standard assay formats for determining cell surface antigens. In general, the antibody is contacted with a sample of the leukocytes to be tested under conditions which allow the antibody to bind IgE-bearing cells in the sample. The cells are then examined for binding of antibody. This can be accomplished by conventional cell staining procedures. For example, a fluorescently labeled second antibody can be used to detect binding of the anti-IgE antibody.

## Claims

1. A peptide representing the ε.mb/ec segment of a dog, the peptide having the sequence shown in SEQ ID NO:3.

2. A peptide having a sequence wherein the sequence shown in SEQ ID NO:3 is modified by deletion, insertion or substitution of one or more amino acids which do not detract from the immunological property of the peptide to elicit antibodies which react specifically with membrane-bound immunoglobulin ε of a dog.

3. A nucleotide consisting of the sequence of the exons (which is the coding portion) of SEQ ID NO:2, or the corresponding or complementary mRNA sequence.

4. Vaccine comprising a peptide having the sequence shown in SEQ ID NO:3, or a peptide having a sequence wherein the sequence shown in SEQ ID NO:3 is modified by deletion, insertion or substitution of one or more amino acids which do not detract from the immunological property of the peptide to elicit antibodies which react specifically with membrane-bound immunoglobulin *ε* of a dog.

5. Use of a peptide having the sequence shown in SEQ ID NO:3 for the preparation of a medicament for treating a dog afflicted with allergy.

6. Use of a peptide having a sequence wherein the sequence shown in SEQ ID NO:3 is modified by deletion, insertion or substitution of one or more amino acids which do not detract from the immunological property of the peptide to elicit antibodies which react specifically with membrane-bound immunoglobulin ε of a dog for the preparation of a medicament for treating a dog afflicted with allergy.

## Patentansprüche

1. Peptid, das das ε.mb/ec-Segment eines Hundes darstellt, wobei das Peptid die in SEQ ID NO:3 gezeigte Sequenz hat.

2. Peptid mit einer Sequenz, in der die in SEQ ID NO:3 gezeigte Sequenz modifiziert ist durch Deletion, Insertion oder Substitution einer oder mehrerer Aminosäuren, die die immunologische Eigenschaft des Peptids, Antikörper, die spezifisch mit membrangebundenem Immunglobulin ε eines Hundes reagieren, zu finden, nicht beeinträchtigen.

3. Nucleotid bestehend aus der Sequenz der Exons (was der codierende Bereich ist) von SEQ ID NO:2 oder der entsprechenden oder komplementären mRNA-Sequenz.

4. Impfstoff aufweisend ein Peptid mit der in SEQ ID NO:3 gezeigten Sequenz oder ein Peptid mit einer Sequenz, in der die in SEQ ID NO:3 gezeigte Sequenz modifiziert ist durch Deletion, Insertion oder Substitution einer oder mehrerer Aminosäuren, die die immunologische Eigenschaft des Peptids, Antikörper, die spezifisch mit membrangebundenem Immunglobulin ε eines Hundes reagieren, zu finden, nicht beeinträchtigen.

5. Verwendung eines Peptids mit der in SEQ ID NO:3 gezeigten Sequenz zur Herstellung eines Arzneimittels zur Behandlung eines an einer Allergie leidenden Hundes.

6. Verwendung eines Peptids mit einer Sequenz, in der die in SEQ ID NO:3 gezeigte Sequenz modifiziert ist durch Deletion, Insertion oder Substitution einer oder mehrerer Aminosäuren, die die immunologische Eigenschaft des Peptids, Antikörper, die spezifisch mit membrangebundenem Immunglobulin ε eines Hundes reagieren, zu finden, nicht beeinträchtigen, zur Herstellung eines Arzneimittels zur Behandlung eines an einer Allergie leidenden Hundes.

## Revendications

1. Peptide représentant le segment ε.mb/ec d'un chien, le peptide ayant la séquence montrée dans SEQ ID NO:3.

2. Peptide ayant une séquence où la séquence montrée dans SEQ ID NO:3 est modifiée par délétion, insertion ou substitution d'un ou plusieurs acides aminés qui ne portent pas atteinte à la propriété immunologique du peptide de déclencher des anticorps qui réagissent spécifiquement avec une immunoglobuline ε liée à la membrane d'un chien.

3. Nucléotide consistant en la séquence des exons ( qui est la partie codante) de SEQ ID NO:2, ou la séquence d'ARNm correspondante ou complémentaire.

4. Vaccin comprenant un peptide ayant la séquence montrée dans SEQ ID NO:3, ou un peptide ayant une séquence où la séquence montrée dans SEQ ID NO:3 est modifiée par délétion, insertion ou substitution d'un ou plusieurs acides aminés qui ne portent pas atteinte à la propriété immunologique du peptide de déclencher des anticorps qui réagissent spécifiquement avec une immunoglobuline ε liée à la membrane d'un chien.

5. Utilisation d'un peptide ayant la séquence montrée dans SEQ ID NO:3 pour la préparation d'un médicament pour traiter un chien souffrant d'allergie.

6. Utilisation d'un peptide ayant une séquence où la séquence montrée dans SEQ ID NO:3 est modifiée par délétion, insertion ou substitution d'un ou plusieurs acides aminés qui ne portent pas atteinte à la propriété immunologique du peptide de déclencher des anticorps qui réagissent spécifiquement avec une immunoglobuline ε liée à la membrane d'un chien pour la préparation d'un médicament pour traiter un chien souffrant d'allergie.


### SEQUENCE LISTING

(1) GENERAL INFORMATION:
   (i) APPLICANT:
      (A) NAME: Tanox Biosystems, Inc.
      (B) STREET: 10301 Stella Link
      (C) CITY: Houston
      (D) STATE: Texas
      (E) COUNTRY: United States of America
      (F) POSTAL CODE (ZIP): 77025
      (G) TELEPHONE: (713)664-2288
      (H) TELEFAX: (713)664-8914
   (ii) TITLE OF INVENTION: Peptides representing antigenic epitopes of IgE present on B cell but not basophil surface
   (iii) NUMBER OF SEQUENCES: 4
   (iv) COMPUTER READABLE FORM:
      (A) MEDIUM TYPE: Floppy disk:
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: EP 93916913.2/0644896
   (vi) PRIOR APPLICATION DATA:
      (A) APPLICATION NUMBER: US 08/031,398
      (B) FILING DATE: 11-MAR-1993
(2) INFORMATION FOR SEQ ID NO: 1:
   (i) SEQUENCE CHARACTERISTICS:
      (A) LENGTH: 14 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO: 1:
   Glu Leu Asp Val Cys Val Glu Glu Ala Glu Gly Glu Ala Pro
   1               5                   10
(2) INFORMATION FOR SEQ ID NO: 2:
   (i) SEQUENCE CHARACTERISTICS:
      (A) LENGTH: 479 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: intron
      (B) LOCATION:283..376
   (ix) FEATURE:
      (A) NAME/KEY: CDS
      (B) LOCATION:148..282
   (ix) FEATURE:
      (A) NAME/KEY: CDS
      (B) LOCATION:377..457
   (xi) SEQUENCE DESCRIPTION: SEQ ID NO: 2:
   AGGTGGCCTG CCACTCCAGC GCCAGCAGGG CTGCCGAGGC CAGGGCATCA TTCTTGTGTC      60
   CCTCGGGGAG GGGGGGCGGG TCTGCCTTCC CCCCACCAGC ACATGAAGCG GCTGGACCGG     120
   GGAGGGCTGA CTGGCCGGTG CCCCCGC GAG CTA GAG CTC CAG GAG CTG TGC         171
                                 Glu Leu Glu Leu Gln Glu Leu Cys
                                  1               5
   GCG GAT GCC ACT GAG AGT GAG GAG CTG GAC GAG CTG TGG GCC AGC CTG       219
   Ala Asp Ala Thr Glu Ser Glu Glu Leu Asp Glu Leu Trp Ala Ser Leu
        10                  15                  20
   CTC ATC TTC ATC ACC CTC TTC CTG CTC AGC GTG AGC TAC GGC GCC ACC       267
   Leu Ile Phe Ile Thr Leu Phe Leu Leu Ser Val Ser Tyr Gly Ala Thr
    25                  30                  35                  40
   AGC ACC CTC TTC AAG GTGGGCATGC AGAGCCCCTG TGCCGGGGGT GGGGGCAGCA       322
   Ser Thr Leu Phe Lys
                    45
   CAGAGGGAGG GAGAGGTCCC GGCAGAGCTA TCCTCACATG TGCCCTCCCC CCAG GTG       379
                                                               Val
                                                                1
   AAG TGG GTA CTC GCC ACC GTC CTG CAG GAG AAG CCA CAG GCC GCC CAA       427
   Lys Trp Val Leu Ala Thr Val Leu Gln Glu Lys Pro Gln Ala Ala Gln
                 5                  10                  15
   GAC TAC GCC AAC ATC GTG CGG CCG GCA CAG TAGGCCCAGA GACACGGTGA         477
   Asp Tyr Ala Asn Ile Val Arg Pro Ala Gln
            20                  25
   CG                                                                    479
(2) INFORMATION FOR SEQ ID NO: 3:
   (i) SEQUENCE CHARACTERISTICS:
      (A) LENGTH: 45 amino acids
      (B) TYPE: amino acid
      (D) TOPOLOGY: linear
   (ii) MOLECULE TYPE: protein
   (xi) SEQUENCE DESCRIPTION: SEQ ID NO: 3:
   Glu Leu Glu Leu Gln Glu Leu Cys Ala Asp Ala Thr Glu Ser Glu Glu
    1               5                  10                  15
   Leu Asp Glu Leu Trp Ala Ser Leu Leu Ile Phe Ile Thr Leu Phe Leu
               20                  25                  30
   Leu Ser Val Ser Tyr Gly Ala Thr Ser Thr Leu Phe Lys
           35                  40                  45
(2) INFORMATION FOR SEQ ID NO: 4:
   (i) SEQUENCE CHARACTERISTICS:
      (A) LENGTH: 27 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Val Lys Trp Val Leu Ala Thr Val Leu Gln Glu Lys Pro Gln Ala Ala
    1               5                  10                  15
   Gln Asp Tyr Ala Asn Ile Val Arg Pro Ala Gln
               20                  25